# EUROPEAN PATENT APPLICATION

(11) **EP 4 628 078 A1**
(43) Date of publication of application: **08.10.2025**
(21) Application number: 23897819.1
(22) Date of filing: 29.11.2023
(51) Int. Cl.: A61K 31/4245, A61P 17/04, A61P 25/00, A61P 43/00

(54) **AGENT FOR PREVENTING OR AMELIORATING ITCH**

(30) Priority: 30.11.2022 JP 2022191756
(71) Applicant: Kao Corporation, Chuo-ku, Tokyo 103-8210 (JP)
(72) Inventor: YOKOTA, Masafumi, Haga-gun, Tochigi 321-3497 (JP); MISAWA, Kensuke, Haga-gun, Tochigi 321-3497 (JP); MATSUMOTO, Tomohiro, Haga-gun, Tochigi 321-3497 (JP); TAKIGUCHI, Kisumi, Haga-gun, Tochigi 321-3497 (JP); MURASE, Daiki, Haga-gun, Tochigi 321-3497 (JP); SUGAI, Yoshiya, Haga-gun, Tochigi 321-3497 (JP)
(74) Representative: Vossius & Partner Patentanwälte Rechtsanwälte mbB
(86) International application number: PCT/JP2023/042721
(87) International publication number: WO 2024/117172

(57) **Abstract**

Provided is a novel material which is useful in the prevention or amelioration of pruritus by inhibiting the C3a receptor signaling pathway. A C3a receptor antagonist comprising, as an active ingredient, ozanimod, a pharmaceutically acceptable salt thereof, or a prodrug thereof.

## Description

### Field of the Invention

The present invention relates to a C3a receptor antagonist, an agent for preventing or ameliorating pruritus, and an agent for preventing or ameliorating a pruritic skin disease.

### Background of the Invention

Pruritus is a clinical condition seen not only in skin diseases such as atopic dermatitis, but also in visceral diseases such as renal failure. Pruritus can also be triggered by dry skin, sunburn, and the rubbing of skin against clothing. The movement of scratching due to pruritus physically injure the skin and cause further aggravation of the symptoms, and therefore the resolution of pruritus contributes to the prevention or amelioration of skin diseases. For example, it has been reported that skin symptoms caused by atopic dermatitis are prevented or ameliorated in mice whose hindlimb nails are cut to suppress physical damage to the skin caused by the movement of scratching (Non Patent Literature 1).

The sensation of pruritus in peripheral tissues such as the skin is transmitted to the brain by afferent sensory nerves which connect the periphery to the dorsal horn of the spinal cord. The cell bodies of afferent sensory nerves reside in the dorsal root ganglia (DRG), and nerve fibers extend from the cell bodies to peripheral tissues and the dorsal horn of the spinal cord. Afferent sensory nerves are responsible for receiving sensations in the skin and transmitting them to secondary neurons in the dorsal horn of the spinal cord.

Pruritogenic substances cause pruritus by binding to the corresponding receptors. Histamine, serotonin, and chloroquine have been reported as pruritogenic substances, and Th2 cytokines such as IL-4 and IL-13 as pruritus-enhancing substances (sensitizers). Of these, a typical chemical pruritogenic substance is histamine, which is mainly secreted by mast cells. Recently, it is considered that histamine is essentially involved in only some acute pruritus, and its involvement in most of the diseases associated with chronic pruritus is poor (Non Patent Literature 2). Therefore, while antihistamines (H1 receptor antagonists) are currently often used as a drug to suppress pruritus, a sufficient therapeutic effect of antihistamines can be obtained against very limited pruritic conditions, and most pruritic conditions are intractable and difficult to remit with antihistamines. Antihistamines here refer to H1 receptor antagonists such as diphenhydramine. For example, the treatment of atopic dermatitis and xeroderma with antihistamines has been reported to be inadequate (Non Patent Literatures 3 and 4), and pruritus associated with many skin diseases such as atopic dermatitis and xeroderma, as well as internal diseases such as renal failure, is considered to be intractable. The mechanism of most cases of intractable pruritus has not been elucidated, and there is a need for the elucidation of the mechanism and the development of novel target molecules.

On the other hand, ozanimod is known as an oral agonist of subtypes 1 and 5 of the sphingosine-1-phosphate (S1P) receptor (Patent Literature 1), and has been reported to possibly decrease the ability of lymphocytes to egress from lymph nodes and reduce the number of circulating lymphocytes in peripheral blood. In Japan, ozanimod is being investigated as an oral therapeutic drug for multiple sclerosis and inflammatory bowel disease.

(Patent Literature 1) JP-A-2011-523412

(Non Patent Literature 1) Hashimoto Y et al. Life Sciences. 2004 Dec 31;76(7):783-94
(Non Patent Literature 2) Ikoma A et al. Nature Reviews Neuroscience. 2006 Jul;7(7):535-47
(Non Patent Literature 3) J Am Acad Dermatol. 2014 Jul; 71(1): 116-132
(Non Patent Literature 4) Future Oncol. 2018 Oct;14(24):2531-2541
(Non Patent Literature 5) Lewis JE et.al. Front Endocrinol (Lausanne). 2015 Feb 2;6:3
(Non Patent Literature 6) Cero C et al Structure. 2014 Dec 2;22(12):1744-1753

### Summary of the Invention

The present invention relates to the following 1) to 3):
1) A C3a receptor antagonist comprising, as an active ingredient, ozanimod, a pharmaceutically acceptable salt thereof, or a prodrug thereof.
2) An agent for preventing or ameliorating pruritus comprising, as an active ingredient, ozanimod, a pharmaceutically acceptable salt thereof, or a prodrug thereof.
3) An agent for preventing or ameliorating a pruritic skin disease comprising, as an active ingredient, ozanimod, a pharmaceutically acceptable salt thereof, or a prodrug thereof.

### Brief Description of Drawings

Figure 1 illustrates the C3a receptor antagonistic effect of ozanimod.
Figure 2 illustrates the results of an RNA-seq analysis of DRG tissue in AEW model mice.
Figure 3 illustrates the results of real-time PCR analysis of Vgf expression level in AEW model mice.
Figure 4 illustrates the results of real-time PCR analysis of Vgf expression level in AD model mice.
Figure 5 illustrates the scratching frequency in C57BL/6J mice after TLQP-21 administration.
Figure 6 illustrates the scratching frequency in mast cell deficient mice after TLQP-21 administration.
Figure 7 illustrates the ameliorating effect on pruritus in AD model mice by the administration of a C3a receptor antagonist.
Figure 8 illustrates the ameliorating effect on pruritus in AEW model mice by the administration of a C3a receptor antagonist.
Figure 9 illustrates the ameliorating effect on pruritus in AD model mice by ozanimod.
Figure 10 illustrates the ameliorating effect on pruritus in ANIT model mice by ozanimod.

### Detailed Description of the Invention

The present inventors first collected dorsal root ganglia (DRG) from model mice showing xeroderma-like skin symptoms after treatment with acetone-ether mixture and water (hereinafter referred to as AEW model mice) and performed a comprehensive gene expression analysis, which identified Vgf as a gene showing differential expression. Furthermore, when quantitative analyses of the Vgf gene expression in DRG of AEW model mice and atopic dermatitis model mice (hereinafter referred to as AD model mice) were performed, Vgf expression in DRG tissue was significantly increased in both model mice compared to control mice (Reference Example 1). Vgf is the gene encoding the neurosecretory factor, VGF nerve growth factor inducible (hereinafter referred to as VGF). VGF is known to undergo degradation by proteases and the like in the organism, which produces several bioactive peptides (Non Patent Literature 5). The injection of one of them, TLQP-21, into the skin of the posterior neck of healthy mice triggered scratching behavior in the mice, and thus TLQP-21 was found to induce pruritus (Reference Example 2). This scratching behavior induced by intradermal TLQP-21 administration was also observed in mast cell-deficient mice, suggesting that TLQP-21 induction of pruritus is a mast cell-independent response (Reference Example 3).

TLQP-21 is known to bind to and elicit signals from the complement factor C3a receptor, which is a GPCR receptor located on the cell membrane (Non Patent Literature 6). Accordingly, in the process of preparing AD and AEW model mice, continuous administration in parallel of a compound which antagonizes the C3a receptor to mice was confirmed to significantly reduce scratching behavior. This demonstrated that the C3a receptor is a target for suppressing pruritus, and that pruritus can be suppressed by inhibiting the binding of TLQP-21 to the C3a receptor (Reference Examples 4 and 5). This suppression of pruritus based on the inhibition of the C3a receptor signaling pathway may be useful as a novel technique for eliminating pruritic conditions for which conventional methods were not effective.

Therefore, the present invention relates to providing a novel material which is useful in the prevention or amelioration of pruritus by inhibiting the C3a receptor signaling pathway.

The present inventors searched for an effective material which inhibits the C3a receptor signaling pathway, and found that ozanimod has a C3a receptor antagonistic effect.

So far, there have been no reports of ozanimod's effect on the C3a receptor and pruritus.

The present invention can prevent or ameliorate pruritus and pruritic skin diseases by inhibiting the C3a receptor signaling pathway.

Ozanimod used in the present invention is 5-[3-[1-(2-hydroxyethylamino)-2,3-dihydro-1H-inden-4-yl]-1,2,4-oxadiazol-5-yl]-2-isopropoxybenzonitrile (CAS Registry Number: 1306760-87-1), of the following formula. Ozanimod has optical isomers, and may be any of the optical isomers or a mixture thereof.

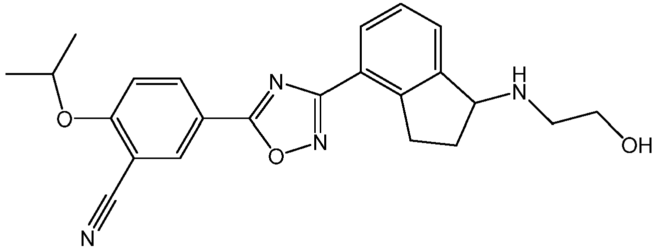

Examples of pharmaceutically acceptable salts of ozanimod include alkali metal salts such as sodium and potassium salts; alkaline earth metal salts such as a calcium salt; a magnesium salt; an aluminum salt; an ammonium salt; organic amine salts such as a trimethylamine salt, a triethylamine salt, a dicyclohexylamine salt, an ethanolamine salt, a diethanolamine salt, a triethanolamine salt, a procaine salt, an N,N'-dibenzylethylenediamine salt, a choline salt, an ethylenediamine salt, a meglumine (N-methylglucamine) salt, a benethamine (N-benzylphenethylamine) salt, a diethylamine salt, a piperazine salt, a tromethamine (2-amino-2-hydroxymethyl-1,3-propanediol) salt, or acid addition salts such as a hydrochloride, a hydrobromide, a sulfate, an acid sulfate, a phosphate, an acid phosphate, a dihydrogen phosphate, an acetate, a succinate, a citrate, a methanesulfonate (mesylate), and a p-toluenesulfonate (tosylate).

Ozanimod may also be made into a prodrug. After being administered to a living organism, a prodrug becomes a pharmaceutically active compound by the action of an enzyme, metabolic hydrolysis, or the like. The prodrug may be any acid derivatives known to those skilled in the art, including, but not limited to, esters produced by the reaction of ozanimod with an appropriate alcohol and amides produced by the reaction of ozanimod with an appropriate amine.

Such ozanimod, pharmaceutically acceptable salt thereof, or prodrug thereof may be a solvate (e.g., hydrate) or a non-solvate.

Ozanimod, the pharmaceutically acceptable salt thereof, or the prodrug thereof is a known compound, and can be produced by a method well known in the art (Patent Literature 1 mentioned above) or obtained by purchasing a commercial product.

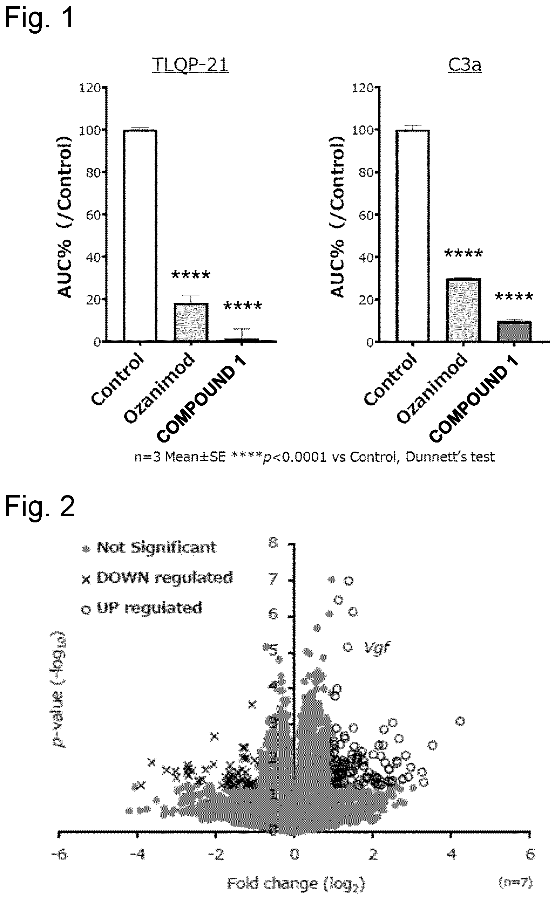

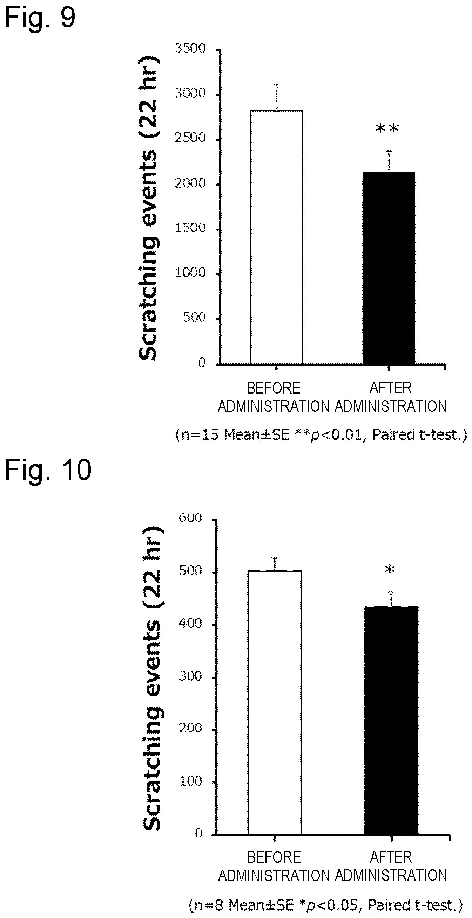

As shown in the Examples described below, ozanimod exhibits C3a receptor antagonism (Figure 1). In addition, a significant decrease in scratching behavior was observed in AD model mice and model mice of pruritus associated with cholestasis (ANIT model mice) which were administered with ozanimod (Figures 9 and 10).

The C3a receptor is a receptor for C3a produced upon complement system activation, and TLQP-21. It has been reported that the inhibition of the C3a receptor signaling pathway suppresses cerebral edema and cerebral hemorrhage induced by a stroke (PLoS One. 2017 Jul 10;12(7):e0180822) and reduces IgG-induced arthritis (J Pharmacol Sci. 2010;112(1):56-63).

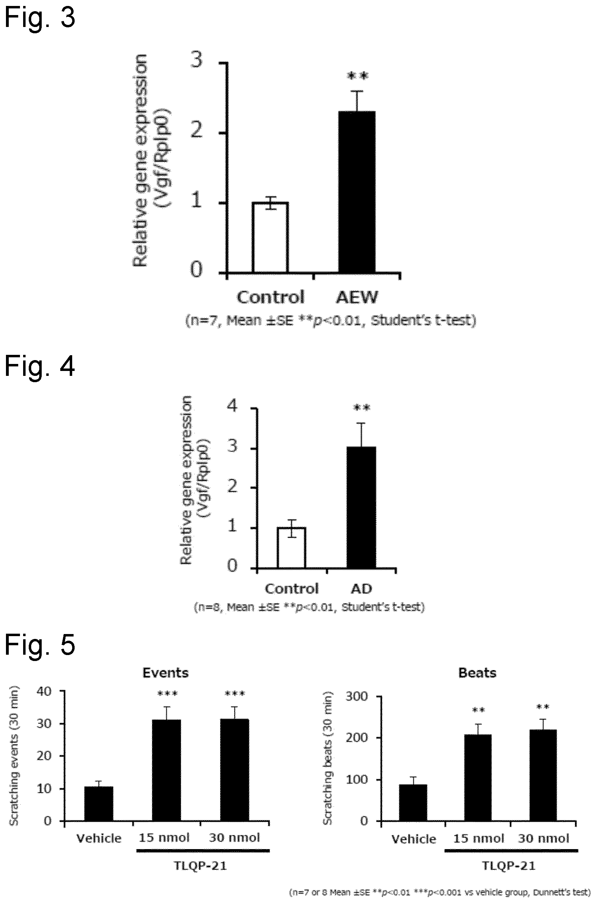

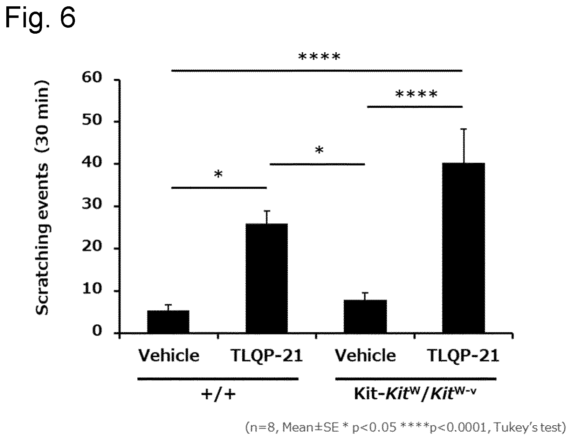

Furthermore, as shown in the reference example 2 below, the injection of TLQP-21, which is produced by the degradation of VGF, into the skin of the posterior neck of healthy mice triggered scratching behavior in the mice (Figure 5). These results clearly show that TLQP-21 induces pruritus. The scratching behavior induced by intradermal TLQP-21 administration was also observed in mast cell-deficient mice (Figure6), which suggests that TLQP-21 induction of pruritus is a mast cell-independent response.

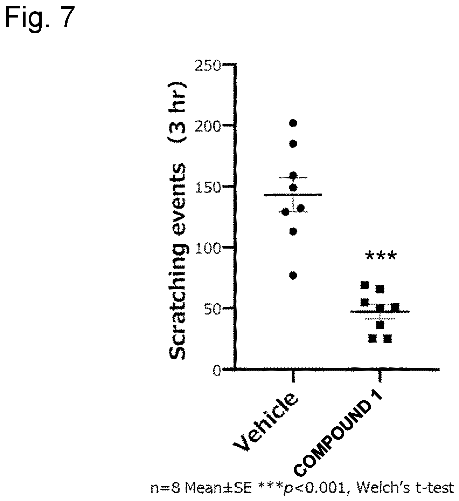

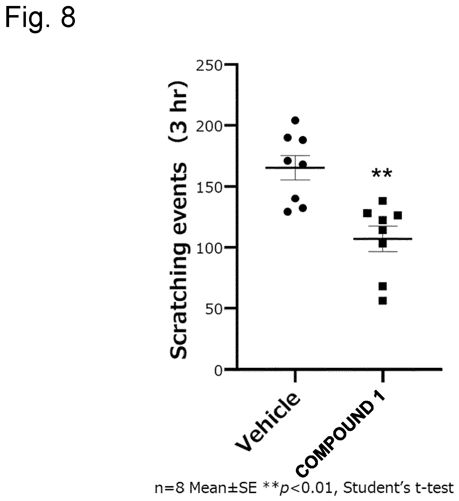

As described above, TLQP-21 is known to bind to the C3a receptor and elicit signals therefrom. Furthermore, when the compound 1, which is known to antagonize the C3a receptor, were continuously administered to mice in the process of preparing AD and AEW model mice, a significant decrease in scratching behavior was observed in the model mice administered with the compound 1 (Figures 7 and 8). This reduction in scratching behavior indicates an alleviating of the pruritus. This clearly shows that C3a receptor is a target for suppressing pruritus, particularly intractable pruritus.

Therefore, the antagonistic inhibition of the binding of TLQP-21 to the C3a receptor by ozanimod allows to prevent or ameliorate pruritus, and to prevent or ameliorate pruritic skin diseases.

That is, ozanimod, a pharmaceutically acceptable salt thereof, or a prodrug thereof (hereinafter also referred to as the "compound of the present invention") can be a C3a receptor antagonist, an agent for preventing or ameliorating pruritus, and an agent for preventing or ameliorating a pruritic skin disease (hereinafter also referred to as "C3a receptor antagonist and the like"), and can be used to produce these agents. The compound of the present invention can also be applied to animals, including humans, to be used to inhibit the C3a receptor signaling pathway, prevent or ameliorate pruritus, and prevent or ameliorate pruritic skin diseases.

Here, "use" can be use in humans or non-human animals, and may be therapeutic or non-therapeutic. "Non-therapeutic" is a concept which does not include medical practices, i.e., a concept which does not include methods of performing surgeries, treatment, or diagnosis on humans, and more specifically, a concept which does not include methods in which a physician or a person directed by a physician performs surgery, treatment, or diagnosis on humans.

In the present description, "C3a receptor antagonism" means the inhibition of the C3a receptormediated action of TLQP-21, and includes, for example, the inhibition of the C3a receptor signaling pathway.

"Pruritus (itch)" is a subjective sensation and its cause is not particularly limited. Examples of the site of pruritus include broad areas and specific sites, such as the entire body, scalp, face, back, arms, back of the hands, fingers, and legs.

The present invention is suitable for the prevention or amelioration of intractable pruritus.

"Intractable pruritus" means pruritus which does not resolve with antihistamines (H1 receptor antagonists). Examples of intractable pruritus include pruritus in diseases such as atopic dermatitis, xeroderma (including senile xerosis, asteatotic dermatitis, and asteatotic eczema), contact dermatitis, seborrheic dermatitis, nummular eczema, psoriasis, prurigo, prurigo nodularis, chronic prurigo, pemphigoid, dermatomyositis, pruritus (e.g., pruritus associated with chronic liver injury, pruritus associated with cholestasis, pruritus associated with chronic kidney injury and the dialysis [hemodialysis or peritoneal dialysis] treatment thereof, senile pruritus, and pruritus hiemalis), and malignant neoplasms. Of these, the intractable pruritus is preferably pruritus in atopic dermatitis, pruritus in xeroderma, pruritus in prurigo nodularis, or pruritus associated with cholestasis.

"Pruritic skin disease" means a skin disease associated with pruritus. Examples thereof include urticaria, atopic dermatitis, xeroderma (including senile xerosis, asteatotic dermatitis, and asteatotic eczema), contact dermatitis, seborrheic dermatitis, nummular eczema, psoriasis, prurigo, prurigo nodularis, chronic prurigo, pemphigoid and dermatomyositis. Of these, with the exception of urticaria, the skin diseases are skin diseases which fall into the category of skin diseases associated with intractable pruritus. The present invention is suitable for the pruritic skin diseases associated with intractable pruritus. The pruritic skin disease is preferably atopic dermatitis, xeroderma, or prurigo nodularis.

In the present description, "prevention" refers to the prevention or delaying of the onset of symptoms in an individual, or the reduction of the risk of onset of symptoms in an individual.

"Amelioration" refers to the reversal, prevention, or delaying of the progression of the worsening of symptoms or condition.

The C3a receptor antagonist and the like of the present invention may itself be a medicament, a quasi-drug, a cosmetic, a food or feed for inhibiting the C3a receptor signaling pathway, preventing or ameliorating pruritus, or preventing or ameliorating a pruritic skin disease, or may be a material or preparation contained in the medicament, quasi-drug, cosmetic, food or feed.

The medicament (including quasi-drug, the same applies hereinafter) contains the compound of the present invention as an active ingredient for inhibiting the C3a receptor signaling pathway, preventing or ameliorating pruritus, or preventing or ameliorating a pruritic skin disease. Furthermore, the medicament may contain a pharmaceutically acceptable carrier, other active or pharmaceutical ingredients, or the like, as needed, as long as the function of the active ingredient is not lost.

The administration of the medicament containing the compound of the present invention can be of any form, and examples thereof include oral and parenteral administration. Examples of dosage forms for oral administration include tablets, capsules, granules, powders, and syrups. Examples of dosage forms for parenteral administration include various preparations such as external skin, transdermal, transmucosal, transnasal, enteral, injectable, suppository, inhaled, and adhesive preparations. For parenteral administration, a suitable form of preparation is an external skin preparation, and specific examples thereof include in the form of an ointment, emulsified liquid, cream, emulsion, lotion, gel, and aerosol.

The cosmetic contains the compound of the present invention as an active ingredient for inhibiting the C3a receptor signaling pathway, for preventing or ameliorating pruritus, or for preventing or ameliorating a pruritic skin disease. Furthermore, the cosmetic may contain a cosmetically acceptable carrier, other active or cosmetic ingredients, or the like, as needed, as long as the function of the active ingredient is not lost.

Preferred examples of cosmetics containing the compound of the present invention include cosmetics for the face and body (e.g., lotions, gels, creams, and facial masks), makeup cosmetics, and cleansers for the face and body.

Each of these medicament and cosmetic preparations can be produced according to a conventional method by combining the compound of the present invention with a pharmaceutically or cosmetically acceptable carrier, the other active, pharmaceutical or cosmetic ingredients described above, and the like, as needed.

Examples of the pharmaceutically or cosmetically acceptable carrier include excipients, diluents, binders, disintegrants, coating agents, solubilizers, lubricant agents, glidants, solubilizing agents, lubricants, various oils, surfactants, gelling agents, pH buffers, tonicity agents, preservatives, antioxidants, solvents, dispersants, chelating agents, thickeners, stabilizers, pH adjusters, pigments, and fragrances.

Examples of the other active, pharmaceutical or cosmetic ingredients include plant extracts, fungicides, moisturizers, anti-inflammatory agents, antibacterial agents, keratolytic agents, ultraviolet absorbers, refrigerants, antiseborrheic agents, cleansing agents, and makeup ingredients.

The food contains the compound of the present invention as an active ingredient for inhibiting the C3a receptor signaling pathway, preventing or ameliorating pruritus, or preventing or ameliorating a pruritic skin disease.

The food includes those with claims of preventing or ameliorating pruritus, or of preventing or ameliorating a pruritic skin disease, and for which labeling to that effect has been approved or notified as necessary (food for specified health uses, food with functional claims, food for special dietary uses, and the like). Examples of labels include "improves skin discomfort (itchy feeling)." Foods for which functional claims are permitted or notified can be distinguished from general foods.

The form of the food can be solid, semi-solid or liquid (e.g., beverage). Examples thereof include various food compositions (breads, cakes, noodles, confectioneries, frozen foods, ice cream, candy, *furikake,* soups, dairy products, shakes, beverages, seasonings, and the like), as well as nutritional supplement compositions in the same forms as the oral preparations described above (solid preparations such as granules, powders, tablets, capsules, microcapsules, and lozenges) .

Foods in various forms can be prepared according to conventional methods by appropriately combining the compound of the present invention with any food material, other active ingredients, or food-acceptable additives (e.g., solvents, softeners, oils, emulsifiers, preservatives, acidulants, sweeteners, bittering agents, pH adjusters, stabilizers, coloring agents, ultraviolet absorbers, antioxidants, moisturizing agents, thickeners, fixing agents, dispersants, fluidity promoters, wetting agents, flavors, seasonings, and flavor modifiers) as desired.

The feed contains the compound of the present invention as an active ingredient for inhibiting the C3a receptor signaling pathway, preventing or ameliorating pruritus, or preventing or ameliorating a pruritic skin disease.

The feed is preferably in the form of pellets, flakes, a mash or liquid, and examples thereof include livestock feed used for cattle, pigs, chickens, sheep, horses, and the like, feed for small animals used for rabbits, rats, mice, and the like, and pet food used for dogs, cats, small birds, and the like.

The feed can be prepared according to conventional methods by appropriately combining the compound of the present invention with other feed materials, such as meat, protein, grains, bran, sake lees, sugars, vegetables, vitamins, minerals, gelling agents, shaperetaining agents, pH adjusters, seasonings, preservatives, and nutritional enhancers.

The content of the compound of the present invention in the above preparation cannot be generally defined, as it differs depending on the form of the preparation and the like, but for example, based on the total amount of the preparation, it is preferably 0.01 mass% or more, more preferably 0.05 mass% or more, and preferably 1.2 mass% or less, more preferably 0.6 mass% or less in terms of ozanimod. In addition, it is preferably from 0.01 to 1.2 mass%, and more preferably from 0.05 to 0.6 mass%.

The dosage or amount of the compound of the present invention used can be an amount which achieves the effect of the present invention. The dosage or amount used can vary according to the species, weight, sex, age, condition of the subject, or other factors, but in the case of oral administration such as of a tablet or capsule, the dosage per adult (60 kg) is preferably 0.01 mg or more, more preferably 0.1 mg or more, and preferably 100 mg or less, more preferably 10 mg or less in terms of ozanimod. In addition, it is preferably from 0.01 mg to 100 mg, and more preferably from 0.1 mg to 10 mg. In the present invention, such amounts can be administered or used repeatedly or continuously for 1 day or more, preferably 7 days or more, more preferably 14 days or more, and even more preferably 42 days or more, divided in one to several doses per day.

Examples of the subject of administration or use of the C3a receptor antagonist and the like of the present invention include humans or non-human animals requiring or desiring the inhibition of the C3a receptor signaling pathway, the prevention or amelioration of pruritus, and the prevention or amelioration of a pruritic skin disease. Specific examples include humans and non-human animals having intractable pruritus or suffering from a pruritic skin disease. Examples of non-human animals include non-human mammals such as anthropoid apes, other primates, and carnivore animals.

The site of administration or use of the C3a receptor antagonist and the like of the present invention is not particularly limited as long as it is a site where pruritus is felt in the case of parenteral administration such as of an external skin preparation.

In relation to the embodiments described above, the present invention further discloses the following aspects.

<1> A C3a receptor antagonist comprising, as an active ingredient, ozanimod, a pharmaceutically acceptable salt thereof, or a prodrug thereof.
<2> An agent for preventing or ameliorating pruritus comprising, as an active ingredient, ozanimod, a pharmaceutically acceptable salt thereof, or a prodrug thereof.
<3> An agent for preventing or ameliorating a pruritic skin disease, comprising, as an active ingredient, ozanimod, a pharmaceutically acceptable salt thereof, or a prodrug thereof.
<4> The agent for preventing or ameliorating pruritus according to <2>, wherein the pruritus is preferably intractable pruritus, more preferably pruritus in diseases such as atopic dermatitis, xeroderma (including senile xerosis, asteatotic dermatitis, and asteatotic eczema), contact dermatitis, seborrheic dermatitis, nummular eczema, psoriasis, prurigo, prurigo nodularis, chronic prurigo, pemphigoid, dermatomyositis, pruritus (e.g., pruritus associated with chronic liver injury, pruritus associated with cholestasis, pruritus associated with chronic kidney injury and the dialysis [hemodialysis or peritoneal dialysis] treatment thereof, senile pruritus, and pruritus hiemalis), and malignant neoplasms, further more preferably pruritus in atopic dermatitis, pruritus in xeroderma, pruritus in prurigo nodularis, or pruritus associated with cholestasis.
<5> The agent for preventing or ameliorating a pruritic skin disease according to <3>, wherein the pruritic skin disease is preferably urticaria, atopic dermatitis, xeroderma (including senile xerosis, asteatotic dermatitis, and asteatotic eczema), contact dermatitis, seborrheic dermatitis, nummular eczema, psoriasis, prurigo, prurigo nodularis, chronic prurigo, pemphigoid or dermatomyositis, and more preferably atopic dermatitis, xeroderma, or prurigo nodularis.
<6> The agent according to any of <1> to <5>, wherein a content of ozanimod, a pharmaceutically acceptable salt thereof, or a prodrug thereof in a preparation is preferably 0.01 mass% or more, more preferably 0.05 mass% or more, preferably 1.2 mass% or less, more preferably 0.6 mass% or less, and preferably from 0.01 to 1.2 mass%, more preferably from 0.05 to 0.6 mass% in terms of ozanimod, based on the total amount of the preparation.
<7> The agent according to any of <1> to <6>, which is a medicament preparation.
<8> The agent according to any of <1> to <7>, wherein a dosage or amount of ozanimod, pharmaceutically acceptable salt thereof, or prodrug thereof used is preferably 0.01 mg or more, more preferably 0.1 mg or more, preferably 100 mg or less, more preferably 10 mg or less, and preferably from 0.01 mg to 100 mg, more preferably from 0.1 mg to 10 mg in terms of ozanimod per dose per adult (60 kg) in the case of oral administration.
<9> Use of ozanimod, a pharmaceutically acceptable salt thereof, or a prodrug thereof for producing a C3a receptor antagonist.
<10> Ozanimod, a pharmaceutically acceptable salt thereof, or a prodrug thereof for use in C3a receptor antagonism.
<11> Non-therapeutic use of ozanimod, a pharmaceutically acceptable salt thereof, or a prodrug thereof for C3a receptor antagonism.
<12> A method for antagonizing a C3a receptor, comprising administering or applying ozanimod, a pharmaceutically acceptable salt thereof, or a prodrug thereof to a subject.
<13> Use of ozanimod, a pharmaceutically acceptable salt thereof, or a prodrug thereof for producing an agent for preventing or ameliorating pruritus.
<14> Ozanimod, a pharmaceutically acceptable salt thereof, or a prodrug thereof for use in preventing or ameliorating pruritus.
<15> Non-therapeutic use of ozanimod, a pharmaceutically acceptable salt thereof, or a prodrug thereof for preventing or ameliorating pruritus.
<16> A method for preventing or ameliorating pruritus, comprising administering or applying ozanimod, a pharmaceutically acceptable salt thereof, or a prodrug thereof to a subject.
<17> The use according to <13>, the ozanimod, the pharmaceutically acceptable salt thereof, or the prodrug thereof according to <14>, the non-therapeutic use according to <15>, or the method according to <16>, wherein the pruritus is preferably intractable pruritus, more preferably pruritus in diseases such as atopic dermatitis, xeroderma (including senile xerosis, asteatotic dermatitis, and asteatotic eczema), contact dermatitis, seborrheic dermatitis, nummular eczema, psoriasis, prurigo, prurigo nodularis, chronic prurigo, pemphigoid, dermatomyositis, pruritus (e.g., pruritus associated with chronic liver injury, pruritus associated with cholestasis, pruritus associated with chronic kidney disease and the dialysis [hemodialysis or peritoneal dialysis] treatment thereof, senile pruritus, and pruritus hiemalis), and malignant neoplasms, further more preferably pruritus in atopic dermatitis, pruritus in xeroderma, pruritus in prurigo nodularis, or pruritus associated with cholestasis.
<18> Use of ozanimod, a pharmaceutically acceptable salt thereof, or a prodrug thereof for producing an agent for preventing or ameliorating a pruritic skin disease.
<19> Ozanimod, a pharmaceutically acceptable salt thereof, or a prodrug thereof for use in preventing or ameliorating a pruritic skin disease.
<20> Non-therapeutic use of ozanimod, a pharmaceutically acceptable salt thereof, or a prodrug thereof for preventing or ameliorating a pruritic skin disease.
<21> A method for preventing or ameliorating a pruritic skin disease, comprising administering or applying ozanimod, a pharmaceutically acceptable salt thereof, or a prodrug thereof to a subject.
<22> The use according to <18>, the ozanimod, the pharmaceutically acceptable salt thereof, or the prodrug thereof according to <19>, the non-therapeutic use according to <20>, or the method according to <21>, wherein the pruritic skin disease is preferably urticaria, atopic dermatitis, xeroderma (including senile xerosis, asteatotic dermatitis, and asteatotic eczema), contact dermatitis, seborrheic dermatitis, nummular eczema, psoriasis, prurigo, prurigo nodularis, chronic prurigo, pemphigoid or dermatomyositis, and more preferably atopic dermatitis, xeroderma, or prurigo nodularis.

### Examples

### Example 1: C3a Receptor Antagonism of Ozanimod

### 1. C3a Receptor Ligand

Mouse TLQP-21 (Tocris Bioscience, sequence: TLQPPASSRRRHFHHALPPAR, the same applies hereinafter) and human C3a recombinant protein (R&D systems) were used. The solvent was HBSS (Gibco).

### 2. Evaluation Sample

The compound 1 (L-arginine, N2-[[5-(diphenylmethyl)-2-thienyl]carbonyl]) synthesized in accordance with a previous report (Rowley, J. A. et al. Journal of Medicinal Chemistry, 2020;63(2):529-541) and ozanimod (Cayman Chemical) were used as C3a receptor antagonists. In the following evaluation, the compound 1 and ozanimod were dissolved in DMSO and diluted with HBSS as appropriate.

### 3. Cells for Evaluating C3a Receptor Response

Human Embryonic Kidney cells 293 (HEK293) were used, into which the expression vectors necessary for evaluation were introduced according to the following. A transfection solution was prepared by sequentially adding human C3a receptor expression vector (OriGene), GNA16 expression vector, and PEI-MAX (PolyScience) solution to serum-free DMEM medium, mixing well, and then allowing it to stand at room temperature for 20 minutes. The prepared transfection solution was added to HEK293 cells seeded in a 6-well cell culture plate and the HEK293 cells were cultured for 24 hours or more to introduce thereto the expression vectors. After culture, the medium was removed, and the cells were washed once with PBS, detached with 0.05% Trypsin/EDTA (Gibco) and subjected to the following Ca²⁺-flux assay. The human GNA16 expression vector was produced by inserting a sequence homologous to the cDNA sequence of human GNA16 (Accession No. M63904.1) referenced by the base sequence database (GenBank) provided by the National Center for Biotechnology Information into the plasmid vector pIRESneo3 (manufactured by Clontech).

### 4. Ca²⁺-Flux Assay

The cells for evaluating C3a receptor response described above were seeded in a BioCoat(Registered Trademark) Poly-D-Lysine 96-well Black Flat Bottom Microplate (CORNING). Calcium Kit-Fluo4 (DOJINDO) was used for the measurement of intracellular Ca²⁺ concentration during C3a receptor stimulation. The Ca²⁺ fluorescent indicator Fluo-4 was incorporated into the cells by adding loading buffer prepared in accordance with the kit manual, and allowing it to stand at 37°C for 1 hour. After washing once with HBSS, recording buffer with the C3a receptor antagonist compound 1 or ozanimod dissolved therein was added. For the control, recording buffer was added. After 15 minutes, C3a receptor ligand solution (mouse TLQP-21 solution or human C3a recombinant protein solution) was added for C3a receptor stimulation, and the Ca²⁺ influx associated with the stimulation was measured over time as intracellular fluorescence intensity (Ex: 480 nm, Em: 540 nm). The value obtained by subtracting the measured value when HBSS was added instead of the C3a receptor ligand solution (blank) was used as the measured value. The compound 1 solution, ozanimod solution, mouse TLQP-21 solution and human C3a recombinant protein solution were added after adjusting their concentrations by appropriately diluting them so that their final concentrations when measuring fluorescence intensity were 1 µM, 10 µM, 1 µM, and 300 nM, respectively. The addition of C3a receptor antagonist solution and C3a receptor ligand solution to the cells for evaluating C3a receptor response and the measurement of fluorescence intensity were performed over time 3 minutes after the addition of the C3a receptor ligand solution, using a fluorescent plate reader equipped with an automatic pipetting function (FDSS/µCELL, Hamamatsu Photonics). Figure 1 shows the results of calculating the integrated value (%) of the fluorescence intensity of ozanimod when the integrated value of the fluorescence intensity measured in the control is set to 100%.

As shown in Figure 1, the fluorescence intensity due to C3a receptor stimulation by C3a receptor ligands measured in the control was suppressed by the addition of the compound 1 or ozanimod, indicating that ozanimod acts as a C3a receptor antagonist.

### Reference Example 1: Comprehensive Gene Expression Analysis

### 1. Experimental Animals

The animals used were male C57BL/6J mice and male NC/Nga mice.
Xeroderma model (AEW model): Absorbent cotton soaked with a 1:1 (v/v) mixture of acetone and diethyl ether was applied on the shaved skin of C57BL/6J mice and allowed to stand for 15 seconds. Immediately thereafter, absorbent cotton soaked with water was applied for 30 seconds. This treatment was repeated twice/day (morning and evening) for 7 days to prepare AEW model mice. Control mice on which only absorbent cotton soaked with water was applied for 30 seconds were prepared as a control. Each group n = 7 was used for the present analysis.

Atopic dermatitis model (AD model): NC/Nga mice were parasitized with mouse fur mites (M. muscuti) to spontaneously generate dermatitis, and were used as an AD model. Mice of the same strain raised in mite-free SPF were used as control mice. Each group n = 8 was used for the present analysis.

### 2. Purification of total RNA

Dorsal root ganglia were excised from the cervical spine of mice. The excised tissues were homogenized by a polytron homogenizer, and total RNA was purified using QIAGEN's RNeasy Mini Kit.

### 3. Gene Expression Analysis

RNA-seq: Total RNA purified from AEW model mice and their control mice was used. SuperScript VILO (Thermo fisher scientific) was used for reverse transcription, and the subsequent treatments followed Thermo fisher scientific's Ion AmpliSeq standard protocol. Ion S5 system was used for sequencing. The results are shown in Figure 2.

As shown in Figure 2, Vgf expression for a given sequencing volume was significantly increased in the AEW model, with p<0.05 by Student's t-test compared to control mice.

Real-Time PCR: Total RNA purified from AEW model mice, AD model mice, and their control mice were used to perform quantitative PCR analysis of the Vgf gene by a Real-Time PCR System using TaqMan Gene Expression Assays specific for Vgf as the target gene. Rplp0 was used as the internal standard. Figures 3 and 4 show the relative expression levels of the Vgf gene when the Vgf gene expression level of control mice is set to 1.

As shown in Figure 3, the Vgf expression level in DRG tissue was significantly increased in AEW model mice compared to control mice. As shown in Figure 4, the Vgf expression level in DRG tissue was also significantly increased in AD model mice compared to control mice.

### Reference Example 2: Expression of Pruritus by TLQP-21

### 1. Experimental Animals

The mice used were male C57BL/6J mice.

### 2. Administration of Candidate Mediator Molecule

Mouse TLQP-21 (Tocris Bioscience) was prepared at concentrations of 15 nmol/20 µL and 30 nmol/20 µL. Saline was used as the solvent. Twenty µL of this was administered into the shaved skin of the posterior neck of C57BL/6J mice. The measurement of the scratching behavior of the mice was started immediately after injection, and the values measured 30 minutes after the start were analyzed.

### 3. Measurement of Scratching Behavior

MicroAct (Neuroscience) was used to measure the scratching behavior after TLQP-21 administration. The results are shown in Figure 5. The total number of a series of continuous scratching movements was indicated as Events, and the total number of scratching movements throughout the measurement period was indicated as Beats.

As shown in Figure 5, after TLQP-21 administration, an increase in the mice's scratching behavior was observed as a significant increase in Events and Beats. The scratching behavior developed as an immediate response within 30 minutes.

### Reference Example 3: TLQP-21-Dependent Expression of Pruritus in Mast Cell-Deficient Mice

### 1. Experimental Animals

WBB6F1/Kit-Kit^{W}/Kit^{W-v} mice were used as mast cell-deficient mice and WBB6F1+/+ mice were used as wild-type control mice. Male mice were used in both cases.

### 2. Administration of Mediator Molecule

Mouse TLQP-21 (Tocris Bioscience) was prepared at a concentration of 20 nmol/20 µL. Saline was used as the solvent. Twenty µL of this was administered into the shaved skin of the posterior neck of WBB6F1/Kit-Kit^{W}/Kit^{W-v} and WBB6F1+/+ mice. The measurement of the scratching behavior of the mice was started immediately after injection, and the values measured 30 minutes after the start were analyzed.

### 3. Measurement of Scratching Behavior

MicroAct (Neuroscience) was used to measure the scratching behavior after TLQP-21 administration. The results are shown in Figure 6. The total number of a series of continuous scratching movements was indicated as Events.

As shown in Figure 6, an increase in mouse scratching behavior due to TLQP-21 administration was observed in both mast cell-deficient and wild-type control mice.

### Reference Example 4: Amelioration of Pruritus in AD Model Mice by C3a Receptor Antagonist

### 1. Experimental Animals

Seven-week-old female NC/Nga mice were used. Preparation of AD model mice: The back of the neck was shaved, and 100 mg of an ointment containing mite allergen: Biostir AD (Biostir Inc.) was applied to the back of the neck and auricular region under isoflurane anesthesia for initial sensitization. Four days after the first application of the ointment containing mite allergen, 150 µL of 4% (w/v) SDS solution and 100 mg of ointment containing mite allergen were applied to the back of the neck and auricular region under isoflurane anesthesia. Thereafter, the same procedure was performed four times at a frequency of once every 3 to 4 days to induce AD-like dermatitis.

### 2. Sample Preparation and Method of Administration

The compound 1, whose C3a receptor antagonism was confirmed in Example 1 above, was used as a C3a receptor antagonist. The compound 1 was dissolved in saline containing 20% polyethylene glycol 400, of which 200 µL was administered subcutaneously to the back of the neck. The control group was administered the same amount of only the above solvent. The concentration of the C3a receptor antagonist solution was set at 0.5 mg/mL. Sample administration started on the day of the second application of the ointment containing mite allergen and was performed three times a week.

### 3. Measurement of Scratching Behavior

Four days after the last application of the ointment containing mite allergen, the scratching behavior was measured for 3 hours after the last administration of the sample. MicroAct (Neuroscience) was used to measure the scratching behavior. The results are shown in Figure 7.

As shown in Figure 7, in AD model mice administered with the compound 1, whose C3a receptor antagonism was confirmed, in parallel with the application of mite allergen, a significant decrease in scratching behavior was observed compared to AD model mice administered with solvent alone.

### Reference Example 5: Amelioration of Pruritus in AEW Model Mice by C3a Receptor Antagonist

### 1. Experimental Animals

The animals used were male C57BL/6J mice. Preparation of AEW model: The back of the neck was shaved, and absorbent cotton soaked with a 1:1 (v/v) mixture of acetone and diethyl ether was applied and allowed to stand for 15 seconds. Immediately thereafter, absorbent cotton soaked with water was applied for 30 seconds. This treatment was repeated twice/day (morning and evening) for 7 days.

### 2. Sample Preparation and Method of Administration

The compound 1, whose C3a receptor antagonism was confirmed in Example 1 above, was used as a C3a receptor antagonist. The compound 1 was dissolved in saline containing 20% polyethylene glycol 400, of which 200 µL was administered subcutaneously to the back of the neck. The control group was administered the same amount of only the above solvent. The concentration of the C3a receptor antagonist solution was set at 0.5 mg/mL. Sample administration started after the first AEW treatment and was performed at a frequency of once daily for 8 days.

### 3. Measurement of Scratching Behavior

Sixteen hours after the last AEW treatment, the scratching behavior was measured for 3 hours after the last administration of the sample. MicroAct (Neuroscience) was used to measure the scratching behavior. The results are shown in Figure 8.

As shown in Figure 8, in AEW model mice administered with the compound 1, whose C3a receptor antagonism was confirmed, in parallel with AEW treatment, a significant decrease in scratching behavior was observed compared to AEW model mice administered with solvent alone.

### Example 2: Amelioration of Pruritus in AD Model Mice by Ozanimod

### 1. Experimental Animals

NC/Nga mice were parasitized with mouse fur mites (M. musculi) to spontaneously generate dermatitis, and were used as an AD model. Fourteen-week-old males having developed dermatitis were used. After one week of acclimation, the back of their necks was shaved and their scratching behavior was measured. The mice were divided into two groups of eight mice each to ensure an equal number of scratching behaviors.

### 2. Preparation of Evaluation Sample

Ozanimod (Selleck Biotech) was used. Carboxymethyl cellulose (Maruishi Pharmaceutical Co., Ltd.) prepared at 0.5% with water for injection was used as a solvent. The weighed ozanimod was lightly crushed using an agate pestle, and then the solvent dropwise addition and mixing were repeated to obtain a suspension (3 mg/mL). The preparation was done on the day of administration and stored at room temperature until administration.

### 3. Administration of Evaluation Sample

The dosage (10 mL/kg) was calculated by measuring body weight, and the prepared evaluation samples were administered orally with an oral gavage needle for mice and a disposable syringe.

### 4. Measurement of Scratching Behavior

MicroAct (Neuroscience) was used to measure the scratching behavior. The number of scratching behaviors was defined as the total number of a series of continuous scratching movements (events), and the measurement time was 22 hours. Measurements were made on the day before (before administration) and on the day of administration (after administration) of ozanimod, and the antipruritic effect of ozanimod was evaluated by comparing the number of scratching behaviors before and after administration. The results are shown in Figure 9.

As shown in Figure 9, a significant decrease in scratching behavior was observed in AD model mice after administration of ozanimod.

Example 3: Amelioration of Pruritus in Model Mice of Pruritus Associated with Cholestasis (ANIT Model Mice) by Ozanimod

### 1. Experimental Animals

Shaved male C57BL/6J mice were used. To eight-week-old C57BL/6J mice, α-naphthyl isothiocyanate (ANIT) dissolved in olive oil (FUJIFILM Wako Pure Chemical) was orally administered once daily for 10 days with an oral gavage needle for mice and a disposable syringe. Before administration, body weight was measured to ensure administration at 25 mg/10 mL/kg (ANIT model). Mice orally administered with ANIT for consecutive days have been reported to exhibit cholestasis-like symptoms and increased scratching behavior (eLife, 2019 8: e44116.).

### 2. Preparation of Evaluation Sample

Ozanimod (Selleck Biotech) was used. Carboxymethyl cellulose (Maruishi Pharmaceutical Co., Ltd.) prepared at 0.5% with water for injection was used as a solvent. The weighed ozanimod was lightly crushed using an agate pestle, and then the solvent dropwise addition and mixing were repeated to obtain a suspension (3 mg/mL). The preparation was done on the day of administration and stored at room temperature until administration.

### 3. Administration of Evaluation Sample

The dosage (10 mL/kg) was calculated by measuring body weight, and the prepared evaluation samples were administered orally with an oral gavage needle for mice and a disposable syringe.

### 4. Measurement of Scratching Behavior

MicroAct (Neuroscience) was used to measure the scratching behavior. The number of scratching behaviors was defined as the total number of a series of continuous scratching movements (events), and the measurement time was 22 hours. Measurements were made on the day before (before administration) and on the day of administration (after administration) of ozanimod, and the antipruritic effect of ozanimod was evaluated by comparing the number of scratching behaviors before and after administration. The results are shown in Figure 10.

As shown in Figure 10, a significant decrease in scratching behavior was observed in ANIT model mice after administration of ozanimod.

## Claims

1. A C3a receptor antagonist comprising, as an active ingredient, ozanimod, a pharmaceutically acceptable salt thereof, or a prodrug thereof.

2. An agent for preventing or ameliorating pruritus comprising, as an active ingredient, ozanimod, a pharmaceutically acceptable salt thereof, or a prodrug thereof.

3. The agent for preventing or ameliorating pruritus according to claim 2, wherein the pruritus is intractable pruritus.

4. The agent for preventing or ameliorating pruritus according to claim 2 or claim 3, wherein the pruritus is pruritus in atopic dermatitis, pruritus in xeroderma, pruritus in prurigo nodularis, or pruritus associated with cholestasis.

5. An agent for preventing or ameliorating a pruritic skin disease, comprising, as an active ingredient, ozanimod, a pharmaceutically acceptable salt thereof, or a prodrug thereof.

6. The agent for preventing or ameliorating a pruritic skin disease according to claim 5, wherein the pruritic skin disease is atopic dermatitis, xeroderma, or prurigo nodularis.

7. Use of ozanimod, a pharmaceutically acceptable salt thereof, or a prodrug thereof for producing a C3a receptor antagonist.

8. Use of ozanimod, a pharmaceutically acceptable salt thereof, or a prodrug thereof for producing an agent for preventing or ameliorating pruritus.

9. The use according to claim 8, wherein the pruritus is intractable pruritus.

10. The use according to claim 8 or 9, wherein the pruritus is pruritus in atopic dermatitis, pruritus in xeroderma, pruritus in prurigo nodularis, or pruritus associated with cholestasis.

11. Use of ozanimod, a pharmaceutically acceptable salt thereof, or a prodrug thereof for producing an agent for preventing or ameliorating a pruritic skin disease.

12. The use according to claim 11, wherein the pruritic skin disease is atopic dermatitis, xeroderma, or prurigo nodularis.

13. Ozanimod, a pharmaceutically acceptable salt thereof, or a prodrug thereof for use in C3a receptor antagonism.

14. Ozanimod, a pharmaceutically acceptable salt thereof, or a prodrug thereof for use in preventing or ameliorating pruritus.

15. The ozanimod, the pharmaceutically acceptable salt thereof, or the prodrug thereof according to claim 14, wherein the pruritus is intractable pruritus.

16. The ozanimod, the pharmaceutically acceptable salt thereof, or the prodrug thereof according to claim 14 or 15, wherein the pruritus is pruritus in atopic dermatitis, pruritus in xeroderma, pruritus in prurigo nodularis, or pruritus associated with cholestasis.

17. Ozanimod, a pharmaceutically acceptable salt thereof, or a prodrug thereof for use in preventing or ameliorating a pruritic skin disease.

18. The ozanimod, the pharmaceutically acceptable salt thereof, or the prodrug thereof according to claim 17, wherein the pruritic skin disease is atopic dermatitis, xeroderma, or prurigo nodularis.

19. Non-therapeutic use of ozanimod, a pharmaceutically acceptable salt thereof, or a prodrug thereof for C3a receptor antagonism.

20. Non-therapeutic use of ozanimod, a pharmaceutically acceptable salt thereof, or a prodrug thereof for preventing or ameliorating pruritus.

21. The non-therapeutic use according to claim 20, wherein the pruritus is intractable pruritus.

22. The non-therapeutic use according to claim 20 or 21, wherein the pruritus is pruritus in atopic dermatitis, pruritus in xeroderma, pruritus in prurigo nodularis, or pruritus associated with cholestasis.

23. Non-therapeutic use of ozanimod, a pharmaceutically acceptable salt thereof, or a prodrug thereof for preventing or ameliorating a pruritic skin disease.

24. The non-therapeutic use according to claim 23, wherein the pruritic skin disease is atopic dermatitis, xeroderma, or prurigo nodularis.

25. A method of C3a receptor antagonism, comprising administering or applying ozanimod, a pharmaceutically acceptable salt thereof, or a prodrug thereof to a subject.

26. A method for preventing or ameliorating pruritus, comprising administering or applying ozanimod, a pharmaceutically acceptable salt thereof, or a prodrug thereof to a subject.

27. The method according to claim 26, wherein the pruritus is intractable pruritus.

28. The method according to claim 26 or 27, wherein the pruritus is pruritus in atopic dermatitis, pruritus in xeroderma, pruritus in prurigo nodularis, or pruritus associated with cholestasis.

29. A method for preventing or ameliorating a pruritic skin disease, comprising administering or applying ozanimod, a pharmaceutically acceptable salt thereof, or a prodrug thereof to a subject.

30. The method according to claim 29, wherein the pruritic skin disease is atopic dermatitis, xeroderma, or prurigo nodularis.
